# EUROPEAN PATENT SPECIFICATION

(11) **EP 3 287 763 B1**
(45) Date of publication and mention of the grant of the patent: **08.05.2019**
(21) Application number: 16813996.2
(22) Date of filing: 03.03.2016
(51) Int. Cl.: G01N 15/02, D21H 21/02, G01N 21/64, G01N 33/34, D21C 9/08

(54) **PITCH ANALYSIS METHOD AND PITCH PROCESSING METHOD**
HARZANALYSEVERFAHREN UND HARZVERARBEITUNGSVERFAHREN
PROCÉDÉ D'ANALYSE DE LA POIX ET PROCÉDÉ DE TRAITEMENT DE LA POIX

(30) Priority: 26.06.2015 JP 2015128596
(43) Date of publication of application: 28.02.2018
(73) Proprietor: Kurita Water Industries Ltd., Tokyo 164-0001 (JP)
(72) Inventor: TAGUCHI, Chigusa, Tokyo 164-0001 (JP); WADA, Satoshi, Tokyo 164-0001 (JP)
(74) Representative: TBK
(86) International application number: PCT/JP2016/056569
(87) International publication number: WO 2016/208225

(56) References cited:
- JP-A- 2007 332 467
- JP-A- 2010 520 444
- JP-A- 2010 529 421
- JP-A- 2014 510 201
- US-A1- 2008 308 241
- US-A1- 2012 258 547
- US-A1- 2013 017 394
- US-A1- 2013 120 556
- US-A1- 2013 220 003
- SEIICHI UCHIDA: "Image processing and recognition for biological images", DEVELOPMENT GROWTH AND DIFFERENTIATION., vol. 55, no. 4, 1 May 2013 (2013-05-01), pages 523-549, XP055459119, US ISSN: 0012-1592, DOI: 10.1111/dgd.12054
- JUNSUKE KAWANA ET AL.: 'DIP no Kyozatsubutsu Hyoka Hoho' DAI 73 KAI KAMI PULP KENKYU HAPPYOKAI 02 June 2006, pages 130 - 133, XP009503173

## Description

### Technical Field

The present invention relates to an analysis method to quantify a pitch in a pulp slurry and measure a particle size distribution of the same, and to a pitch processing method using this analysis method.

### Background Art

In a paper manufacturing process, among contaminants included in a pulp, i.e., a raw material of paper, a sticky hydrophobic substance derived from a wooden material and a used paper is called a pitch. This pitch is present in a dispersion state as colloidal microparticles in a pulp slurry, wherein this pitch aggregates in a preparation process and a paper making process to form a coarse pitch, which causes deterioration in quality of a paper product, poor squeezing out of water, breakage of a paper, and so forth, and this in turn causes a trouble in operation. The pitch trouble like this tends to occur especially when used papers such as a deinked pulp and a carton box paper as well as a waste paper are reused as raw materials.

Therefore, in order to avoid the pitch trouble, before the pulp slurry is sent to a paper making machine it is preferable to remove the pitch or to suppress aggregation of the pitch; and thus, usually a pitch controlling agent is added into the pulp slurry in advance. Further, in order to surely avoid the pitch trouble, it is required to effectively add the pitch controlling agent so as not to form the coarse pitch.

For this, it is important to accurately quantify the pitch in the pulp slurry.

As to a conventional quantification method of the pitch, for example, in Patent Literature 1, a method is disclosed that after a pitch (hydrophobic organic particles) in a filtrate after a filtration process of the pulp slurry is dyed with a fluorescent dye, a fluorescence strength is measured so as to measure a mass concentration of the pitch in the filtrate.

In Patent Literature 2, it is described that upon filtering a sample obtained after the fluorescence measurement, a size of the pitch (hydrophobic contaminants) is determined in accordance with a pore diameter of a filter used in this filtration.

On the other hand, Patent Literatures 3 and 4 describe a method using an image analysis wherein a sticky material on residues after filtration is heat-treated under pressure, and then it is transcribed and attached onto a medium such as a filter paper and a stainless steel so as to use it as a sample for the image analysis.

Further, Non-Patented Literature 1 describes a method for separating the pitch from fibers, ashes, and the like wherein a fiber classification analysis apparatus of a tube flow type is used.

US2012258547 (A1) relates to a device and method to perform real-time macrostickies and/or any visible hydrophobic particle analysis in an aqueous medium, wherein the technique is based on fluorescence image analysis to identify and count sticky particles as well as measure their size.

Uchida, S. (2013, Image processing and recognition for biological images. Develop. Growth Differ., 55: 523-549. doi:10.1111/dgd.12054), reviews image processing and pattern recognition techniques, which will be useful to analyze bioimages.

US2013120556 (A1) relates to an analyser for a pulp and/or a white water stream and to a method of analysis of macrocontaminants, the method comprising: separating a sample from the stream into a fraction of macrocontaminants; producing at least one detected image by optical measurement of the fraction; and analysing the at least one detected image and determining the quantity and type of at least one macrocontaminant in the fraction.

US2013220003 (A1) relates to a method and an apparatus for measuring the depositability of particulate contaminants present in a pulp or paper mill fluid stream, wherein the amount of contaminants collected on the substrate is quantified and evaluated by taking one or more scanned images of the substrate with a resolution of at least 2,000 dots per inch (DPI) and analyzing the scanned images with image analysis technique.

JP2007332467 (A) relates to a method for objectively analyzing and quantifying the number, size, shape, etc., of particles containing colloidal pitch in a papermaking system, comprising selecting a specific color component of a microscopic image of fibers and particles containing pitch particles in a papermaking raw material liquid before papermaking or white liquor on a computer screen by image analyzing software, extracting the fibers and particles containing the pitch particles, displaying the fibers and particles on the screen and thereby analyzing and quantifying the pitch particles.

US2013017394 (A1) relates to a method to produce on a commercial scale, high aspect ratio cellulose nanofilaments (CNF) from natural lignocellulosic fibers comprises a multi-pass high consistency refining (HCR) of chemical or mechanical fibers using combinations of refining intensity and specific energy.

### Citation List

### Patent Literatures

Patent Literature 1: Japanese Patent Application Publication No. 2010-520444
Patent Literature 2: Japanese Patent Application Publication No. 2012-521009
Patent Literature 3: Japanese Patent Laid-Open Publication No. 2007-271389
Patent Literature 4: Japanese Patent No. 4802156

### Non-Patent Literature

Non-Patent Literature 1: Moe Fukuoka (plus three other authors) "Study on the analysis of a pulp slurry using a novel fiber classification analysis apparatus (part 2)", [online], March 16, 2015, Internet <DOI: http://doi.org/10.2524/jtappij.1503>

### Summary of Invention

### Technical Problems

As described above, various methods have been proposed for observation and quantification of the pitch. However, as described in Patent Literatures 3 and 4, when the quantitative analysis is carried out by using the sample which is transcribed onto the medium by making use of the pitch's stickiness, at odd times the substance attached onto the medium by this transcription is a mixture of foreign materials such as fine fibers and calcium carbonate that are intervened with the pitch; and thus, it is difficult to say that the quantification is made only for the pitch.

On the other hand, according to the measurement method of fluorescence by way of dyeing the pitch, only the hydrophobic pitch can be separated so as to be quantified. However, in the measurement of a scattered light or a transmitted light by means of a fluorescence spectrophotometer such as those described in Patent Literatures 1 and 2, the relationship between the fluorescence strength and the concentration is influenced by the particle size; and thus, it is difficult to say that this is the method with which an accurate quantification can be carried out. On top of it, in an actual operation, the relationship between the pitch trouble and the fluorescence strength is not clear.

Further, even when the pitch in the filtrate is measured in the way as described in Patent Literature 1, the coarse pitch that causes the pitch trouble cannot be detected.

When an apparatus such as the fiber classification analysis apparatus of a tube flow type described in Non-Patent Literature 1 is used, the pitch can be observed in the state that it is separated from fibers; however, because the pitch is unstable in water, if the time to start the analysis takes too long, an accurate analysis cannot be carried out because of chronological change of a state of the pitch.

Accordingly, the method with which a state of the pitch in water can be known more accurately and conveniently has been wanted.

The present invention was made under the circumstances as mentioned above; and thus, the present invention has an object to provide the pitch analysis method with which the pitch in water can be quantified not only promptly and conveniently but also more accurately in accordance with the particle size thereof. In addition, the present invention also has an object to provide the pitch processing method wherein in a paper making process, a pitch controlling agent is effectively added by using this analysis method.

### Solution to Problems

The analysis method of the present invention uses an image analysis as a means to accurately quantify the pitch in water, wherein by way of this method, a pitch controlling agent can be effectively added in a paper making process.

That is, the present invention provides the following [1] to [3].
[1] A pitch analysis method, wherein a pulp slurry including a pitch that is dyed with a hydrophobic fluorescent dye added into a pulp slurry is observed with a fluorescence microscope to obtain an observation image; the observation image thus obtained is subjected to an image analysis by using an image analysis software; and by way of this image analysis, quantification of the pitch and measurement of a particle size distribution of the pitch are carried out, wherein the image analysis comprises following (1) to (4) processes to be carried out in this sequence:
   (1) a conversion process to convert a color image to a gray scale image;
   (2) a binarization process to binarize the gray scale image into white and black after removing noises in the gray scale image;
   (3) a removal process to remove regions of particle portions with an aspect ratio of 0.2 or less in a white region of an image after the binarization process, wherein the aspect ratio means the ratio of the length of the short axis of the particle portion in the image to the length of the long axis of the same (short axis/long axis); and
   (4) a statistical process in which an area of each particle in the white region of the image after the removal process is measured, and then, particle size distribution of each area fraction is obtained.
[2] The pitch analysis method according to [1], wherein in the binarization process, with regarding the black as zero and the white as 100, the binarization is made such that a region where a brightness of the gray scale image is equal to or less than a predetermined value V be regarded as the black and a region where the brightness is more than V be regarded as the white, and this V is in a range of 10 to 50.
[3] A pitch processing method, wherein the pitch analysis method according to [1] or [2] is carried out in a predetermined spot in a paper making system, and then, a pitch controlling agent is added to a spot where a pitch having an area equal to or greater than a predetermined area is present in the image.

### Advantageous Effects of Invention

According to the pitch analysis method of the present invention, a pitch in water can be quantified not only promptly and conveniently but also more accurately in accordance with the particle size thereof. Therefore, by using the analysis method of the present invention, the spot where many of the coarse pitch to cause a pitch trouble are distributed can be promptly determined in a paper making process.

Therefore, according to the pitch processing method of the present invention, the optimum spot where a pitch controlling agent to suppress the pitch trouble is added can be determined; and thus, the pitch controlling agent can be effectively added.

### Description of Embodiments

Hereunder, embodiments of the present invention will be explained in detail.

### [Pitch Analysis Method]

The present invention is the pitch analysis method, wherein a pulp slurry including a pitch that is dyed with a hydrophobic fluorescent dye added into a pulp slurry is observed with a fluorescence microscope to obtain an observation image; the observation image thus obtained is subjected to an image analysis by using an image analysis software; and by way of this image analysis, quantification of the pitch and measurement of a particle size distribution of the pitch are carried out.

The image analysis comprises following (1) to (4) processes to be carried out in this sequence:
(1) a conversion process to convert a color image to a gray scale image;
(2) a binarization process to binarize the gray scale image into white and black after removing noises in the gray scale image;
(3) a removal process to remove regions of particle portions with an aspect ratio of 0.2 or less in a white region of an image after the binarization process, wherein the aspect ratio means the ratio of the length of the short axis of the particle portion in the image to the length of the long axis of the same (short axis/long axis); and
(4) a statistical process in which an area of each particle in the white region of the image after the removal process is measured, and then, particle size distribution of each area fraction is obtained.

The analysis method mentioned above is carried out for image observation by selectively dyeing the pitch in a pulp slurry, whereby the pitch can be quantified in accordance with the particle size thereof without requiring an operation such as filtration, so that, accuracy of measurement of the particle size distribution can be improved, especially even in a coarse pitch.

In addition, because the sample for analysis can be observed and measured immediately after sampling, an effect of the chronological change of the pitch can be suppressed. Therefore, a state of the pitch in a paper making process can be known more accurately; and thus, the spot in which many of the coarse pitch to cause the pitch trouble are distributed can be promptly determined.

### (Dyeing)

The fluorescent dye added into the pulp slurry is for dyeing the pitch. The pitch is a hydrophobic sticky substance, whereas a pulp fiber, which is mainly composed of cellulose, is hydrophilic. Therefore, by using a hydrophobic dye, the pitch can be selectively dyed in the pulp slurry. Specific example of the hydrophobic dye for dyeing the pitch includes, among others, Fluorol 555, Nile Red, and an Acridine Orange. Among these dyes, in view of easy observation, the dye which can express a strong contrast is preferable, so that Fluorol 555 having a yellowish orange color is preferably used.

In view of prompt dyeing, it is preferable that the hydrophobic dye be added into the pulp slurry after it is dissolved into an organic solvent. As to the organic solvent, in order to promptly disperse into an aqueous system of the pulp slurry, a hydrophilic organic solvent is preferably used. Therefore, for example, ethanol, propanol, tetrahydrofuran, and the like are preferably used, while especially ethanol is preferably used.

Addition amount of the fluorescent dye is not particularly restricted, so that the amount is arbitrarily controlled in accordance with the pitch content in the pulp slurry. Therefore, the concentration and addition amount thereof are sufficient if these are roughly in a level at which dyeing of the pitch can be satisfactorily confirmed visually, so that the amount more than necessary is not preferable for image observation. Therefore, normally, the addition is made such that the dye concentration may be in the range of 0.001 to 10 mg/L.

### (Observation using fluorescence microscope)

The pitch that is dyed with a fluorescent dye can be observed more clearly by using a fluorescence microscope.

This observation image is preferably photographed for image analysis by means of a CCD camera, a digital camera, or the like.

When the magnification and image size of the microscope are larger, the accuracy thereof increases as well; but these are not particularly restricted. Therefore, it is satisfactory when they are roughly in a level capable of carrying out the analysis process in the image analysis to be carried out thereafter.

### (Image analysis)

The image analysis of the observation image thus photographed is carried out by using an image analysis software on a computer. In the image analysis, the (1) to (4) processes mentioned before are carried out in this sequence. Hereunder, the processes (1) to (4) will be explained in this sequence.

### (1) Conversion process

To begin with, the color image of the pitch that is dyed with a fluorescent dye is converted to a gray scale image. Namely, in the image analysis of the present invention, the elements of hue and saturation in the image of the dyed pitch (analysis sample) are removed in advance so as to make it non-colored.

### (2) Binarization process

Next, noises included in the gray scale image obtained in (1) are removed so as to binarize it to white and black. Substances that are dyed with the fluorescent dye other than the pitch as well as the pitch overlapped with an image plane in the upper and lower sides thereof are detected with low brightness (whiteness) in the gray scale image. Therefore, in order to accurately quantify the pitch, it is preferable to remove these noises. Further, in order to make the analysis easy, the region where the pitch is present is made the mono-colored white region, while the region other than this region is made to the black region.

Specifically, in the image analysis software, with regarding the black as zero and the white as 100, the binarization is made such that the region where a brightness of the gray scale image is equal to or less than a predetermined value V be regarded as the black and the region where the brightness is more than V be regarded as the white. Namely, the binarization is made such that the region where the brightness is equal to or less than V be regarded as the noise, and only the region where the brightness is more than V be regarded as the white (mono-colored), and further, the region other than this region be regarded as the black (mono-colored).

The value V is arbitrarily determined in accordance with resolution of the image, characteristics of the image analysis software, and the like, wherein the value V is preferably in the range of 10 to 50, more preferably in the range of 15 to 40, while still more preferably in the range of 20 to 30.

When the noises are removed by way of setting the threshold as mentioned above, the pitch can be detected more accurately.

### (3) Removal process

The regions of particle portions with an aspect ratio of 0.2 or less in the white region of an image after the binarization process (2) are removed. Meanwhile, the aspect ratio mentioned in the present invention means the ratio of the length of the short axis of the particle portion in the image to the length of the long axis of the same (short axis/long axis).

It is usually presumed that a long and narrow particle portion with the aspect ratio of 0.2 or less is not the pitch but a fibrous contaminant that is dyed with the fluorescent dye. Therefore, in order to secure an accurate detection of the pitch, it is preferable to carry out quantification of the pitch after the particle portions having the aspect ratio like this are removed.

### (4) Statistical process

An area of each particle in the white region of the image after the removal process (3) is measured, and then, particle size distribution of each area fraction is obtained. By way of the process like this, when the pitch is quantified from the particle area, not only the particle size distribution of the pitch can be obtained promptly and conveniently without including contaminants and noises other than the pitch, but also the accuracy of the statistical process can be improved.

Meanwhile, the image analysis software to be used in the present invention is not particularly restricted so far as it can carry out the image analysis processes of (1) to (4). For example, WinROOf (manufactured by Mitani Corp.) and the like can be suitably used.

### [Pitch processing method]

When the pitch is analyzed in a predetermined spot in a paper making system by way of the pitch analysis method of the present invention, the spot where a coarse pitch having an area larger than a predetermined area as well as the amount thereof can be easily determined from the obtained particle size distribution data of the pitch. The area mentioned here means the area of the pitch measured from the observation image in the pitch analysis method mentioned above.

The predetermined area can be arbitrarily set in accordance with the kind and quality of the paper to be manufactured, characteristics of the paper making machine, and so forth; however, for example, the area of 700 µm² or more may be regarded as the coarse pitch so as to predetermine the spot where distribution of the coarse pitch is seen. By adding the pitch controlling agent to the spot that is determined as mentioned above, formation of the coarse pitch can be effectively suppressed.

Accordingly, because the effective addition spot of the pitch controlling agent can be determined in the paper making system, the pitch in the paper making process can be effectively controlled by the pitch controlling agent.

### EXAMPLES

Hereunder, the embodiments of the present invention will be explained more specifically; but the present invention is not limited by Examples shown below.

### (Test 1) Quantitative test of the pitch

Into the slurry of the broad-leaved tree bleached kraft pulp (LBKP; Canadian Freeness Standard (C. F. S.): 200 mL) prepared such that the concentration of suspended solids (SS) in water might become 1% by mass, was added as a simulation pitch 100 mg/L (relative to the slurry) of water-based acryl-type adhesive Regitex A-6001 (manufactured by Regitex Inc.). Into 2 mL of this analysis sample of the pulp slurry was added an ethanol solution containing 0.1% by mass of hydrophobic fluorescent dye (Fluorol 555, manufactured by Exciton, Inc.) such that the dye concentration might become 0.01 mg/L; and then, a resulting mixture was stirred by using a vortex mixer. The sample thus dyed (10 µL) was promptly taken by using a micropipette for the use of 200 µL, and then, it was observed with a fluorescence microscope under the condition shown below; and the photograph thereof was taken by a CCD camera.
Microscope: fluorescence mirror unit U-FBW, manufactured by Olympus Corp.
Fluorescent light source: halogen lamp U-HGLGPS, manufactured by Olympus Corp.
CCD camera: DP73-SET-A, manufactured by Olympus Corp.
   Eyepiece: magnification of 10
   Objective: magnification of 10
   Shutter speed: 1 second
   Field stop and aperture stop: both open
   Halogen lamp strength: 3
   Image size: 1200×1600 pixel

The image data thus obtained was subjected to the image analysis by using the image analysis measurement software (WinROOF, manufactured by Mitani Corp.). The analysis process comprising the following (1) to (4) was carried out in this sequence:
(1) a conversion process to convert a color image to a gray scale image;
(2) a binarization process to binarize the gray scale image such that, with regarding the black as zero and the white as 100, a region where a brightness of the gray scale image is equal to or less than 20 be regarded as the black and the region where the brightness is more than 20 be regarded as the white;
(3) a removal process to remove regions of particle portions with an aspect ratio of 0.2 or less in a white region of an image after the binarization process; and
(4) a statistical process in which an area of each particle in the white region of the image after the removal process is measured, and then, particle size distribution of each area fraction is obtained.

In Table 1 below, the particle size distributions with and without carrying out the removal process (3) are shown.

**[Table 1]**

| Unit: µm² | | Total area of each area fraction | | | | | | | | Grand total area |
|---|---|---|---|---|---|---|---|---|---|---|
| Area fraction | | 0 to less than 5 | 5 to less than 10 | 10 to less than 100 | 100 to less than 200 | 200 to less than 500 | 500 to less than 700 | 700 to less than 1000 | 1000 or more | |
| Treatment (3) | Yes | 1570 | 704 | 3520 | 1257 | 930 | 1137 | 749 | 0 | 9867 |
| | No | 1617 | 743 | 4096 | 1504 | 930 | 1137 | 749 | 24478 | 35255 |

As can be seen from the results in Table 1, it was confirmed that the pitch can be quantified by image analysis of the observation image using the fluorescence microscope.

In addition, by carrying out the removal process (3), long and narrow particle portions (fiber portions) that are visually observable are removed; especially, the particle portions in the area fraction of 1000 µm² or more in the measured particle size distribution are removed. From this, it can be said that quantification of only the pitch and measurement of the fractionated particle size can be conveniently carried out without including the fibers in the pulp slurry.

### (Test 2) Application test of the pitch controlling agent (1)

By using, as the raw materials, the waste papers in the carton box producing process and used papers of carton boxes, the pitch in a 5-layer cardboard-making machine to produce a jute liner was analyzed by using the same analysis method as mentioned before (Test 1). In the paper making process to produce the surface layer, a pitch controlling agent (Spanplus 520, manufactured by Kurita Water Industries Ltd.) was added into a mixing tank in the paper making system with the addition amount of 500 g per 1 ton of the paper production amount.

The analysis sample of the pulp slurry each was taken from a machine tank, a mixing tank, an inlet, and a white water silo, respectively. The image analysis according to the (1) to (4) analysis processes in Test 1 was carried out at 5 spots in the observation image for each sample. The average value was used in quantification of the pitch and measurement of the fractionated particle size.

In Table 2 below, the particle size distributions of the pitch are shown.

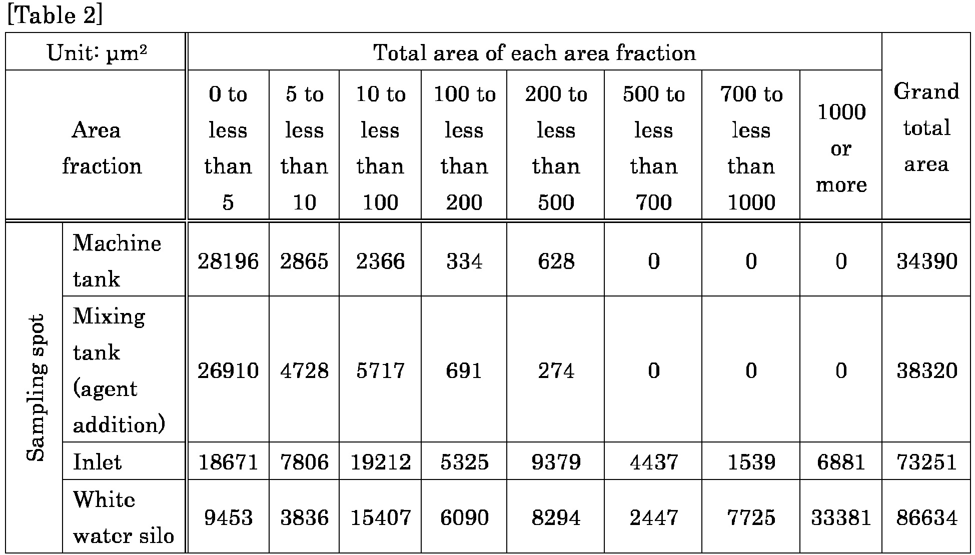

As can be seen from the results in Table 2, it was found that there are many pitches in the inlet and the white water silo, and also that many pitches with the area fractions of 700 µm² or more are distributed especially in the inlet and the white water silo.

### (Test 3) Application test of the pitch controlling agent (2)

The same procedure to carry out the pitch analysis as mentioned before (Test 2) was followed, except that, in Test 2, the addition spot of the pitch controlling agent was changed to the inlet.

In Table 3 below, the particle size distributions of the pitch are shown.

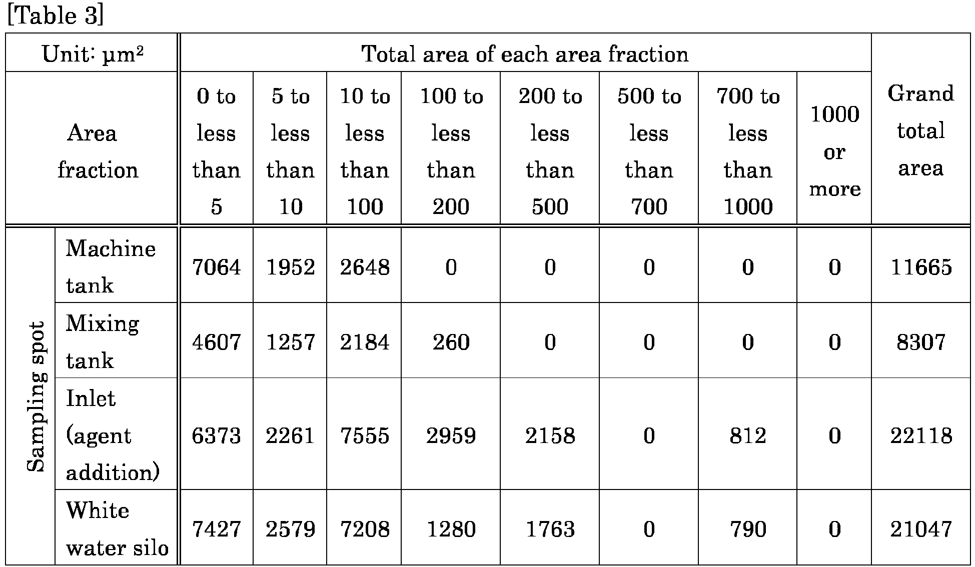

As can be seen from the results in Tables 2 and 3, when the addition spot of the pitch controlling agent was changed to the inlet where many pitches with the area fractions of 700 µm² or more were distributed in Test 2, the pitch having relatively large particle size (area fraction) was decreased in the entire paper making system. From this, it can be said that according to the analysis method of the present invention, in order to reduce the coarse pitch in the paper making system, a more effective addition spot of the pitch controlling agent can be determined.

## Claims

1. A pitch analysis method, wherein a pulp slurry including a pitch that is dyed with a hydrophobic fluorescent dye added into a pulp slurry is observed with a fluorescence microscope to obtain an observation image; the observation image thus obtained is subjected to an image analysis by using an image analysis software; and by way of this image analysis, quantification of the pitch and measurement of a particle size distribution of the pitch are carried out, wherein the image analysis comprises following (1) to (4) processes to be carried out in this sequence:
(1) a conversion process to convert a color image to a gray scale image;
(2) a binarization process to binarize the gray scale image into white and black after removing noises in the gray scale image;
(3) a removal process to remove regions of particle portions with an aspect ratio of 0.2 or less in a white region of an image after the binarization process, wherein the aspect ratio means the ratio of the length of the short axis of the particle portion in the image to the length of the long axis of the same (short axis/long axis); and
(4) a statistical process in which an area of each particle in the white region of the image after the removal process is measured, and then, particle size distribution of each area fraction is obtained.

2. The pitch analysis method according to claim 1, wherein in the binarization process, with regarding the black as zero and the white as 100, the binarization is made such that a region where a brightness of the gray scale image is equal to or less than a predetermined value V be regarded as the black and a region where the brightness is more than V be regarded as the white, and this V is in a range of 10 to 50.

3. A pitch processing method, wherein the pitch analysis method according to claim 1 or claim 2 is carried out in a predetermined spot in a paper making system, and then, a pitch controlling agent is added to a spot where a pitch having an area equal to or greater than a predetermined area is present in the image.

## Patentansprüche

1. Harzanalyseverfahren, wobei eine Zellstoffaufschlämmung, die ein Harz beinhaltet, das mit einem zu einer Zellstoffaufschlämmung zugegeben hydrophoben Fluoreszenzfarbstoff gefärbt ist, mit einem Fluoreszenzmikroskop beobachtet wird, um ein Beobachtungsbild zu erhalten; das so erhaltene Beobachtungsbild einer Bildanalyse unter Verwendung einer Bildanalysesoftware unterzogen wird; und mittels dieser Bildanalyse die Quantifizierung des Harzes und die Messung einer Teilchengrößenverteilung des Harzes durchgeführt werden, wobei die Bildanalyse folgende (1)- bis (4)-Prozesse umfasst, die in dieser Reihenfolge durchzuführen sind:
(1) ein Konvertierungsprozess zum Konvertieren eines Farbbildes in ein Graustufenbild;
(2) einen Binarisierungsprozess, um das Graustufenbild nach dem Entfernen von Rauschen im Graustufenbild in Weiß und Schwarz zu binarisieren;
(3) ein Entfernungsprozess zum Entfernen von Bereichen von Teilchenabschnitten mit einem Seitenverhältnis von 0,2 oder weniger in einem weißen Bereich eines Bildes nach dem Binarisierungsprozess, wobei das Seitenverhältnis das Verhältnis der Länge der kurzen Achse des Teilchenabschnitts im Bild zur Länge der langen Achse derselben (kurze Achse/lange Achse) bedeutet; und
(4) einen Statistikprozess, bei dem eine Fläche jedes Teilchens im weißen Bereich des Bildes nach dem Entfernungsprozess gemessen wird und dann die Teilchengrößenverteilung jeder Flächenfraktion erhalten wird.

2. Harzanalyseverfahren nach Anspruch 1, wobei im Binarisierungsprozess, mit Bezug auf das Schwarz als Null und das Weiß als 100, die Binarisierung so durchgeführt wird, dass ein Bereich, in dem eine Helligkeit des Graustufenbildes gleich oder kleiner als ein bestimmter Wert V ist, als das Schwarz, und ein Bereich, in dem die Helligkeit größer als V ist, als das Weiß betrachtet wird, und dieses V in einem Bereich von 10 bis 50 liegt.

3. Harzverarbeitungsverfahren, wobei das Harzanalyseverfahren nach Anspruch 1 oder Anspruch 2 an einer bestimmten Stelle in einem Papierherstellungssystem durchgeführt wird, und dann ein Harzsteuerungsmittel an einer Stelle hinzugefügt wird, an der ein Harz mit einer Fläche gleich oder größer als eine bestimmte Fläche im Bild vorhanden ist.

## Revendications

1. Procédé d'analyse de la poix, dans lequel une pâte liquide incluant une poix qui est colorée avec un colorant fluorescent hydrophobe ajouté dans une pâte liquide est observée avec un microscope à fluorescence pour obtenir une image d'observation ; l'image d'observation ainsi obtenue est soumise à une analyse d'images en utilisant un logiciel d'analyse d'images ; et au moyen de cette analyse d'images, une quantification de la poix et une mesure de la distribution granulométrique de la poix sont mises en oeuvre, dans lequel l'analyse d'images comprend les processus (1) à (4) suivants à mettre en oeuvre dans cet ordre :
(1) un processus de conversion pour convertir une image colorée en une image à niveaux de gris ;
(2) un processus de binarisation pour binariser l'image à niveaux de gris en blanc et noir après élimination des bruits dans l'image à niveaux de gris ;
(3) un processus d'élimination pour éliminer des régions de parties particulaires avec un rapport de forme de 0,2 ou moins dans une région de blanc d'une image après le processus de binarisation, dans lequel le rapport de forme signifie le rapport entre la longueur de l'axe court de la partie particulaire dans l'image et la longueur de l'axe long de celle-ci (axe court/axe long) ; et
(4) un processus statistique dans lequel une zone de chaque particule dans la région de blanc de l'image après le processus d'élimination est mesurée, puis, une distribution granulométrique de chaque fraction de zone est obtenue.

2. Procédé d'analyse de la poix selon la revendication 1, dans lequel dans le processus de binarisation, en considérant le noir comme étant zéro et le blanc comme étant 100, la binarisation est réalisée de sorte qu'une région où une luminosité de l'image à niveaux de gris est égale ou inférieure à une valeur prédéterminée V soit considérée comme le noir et une région où la luminosité est supérieure à V soit considérée comme le blanc, et ce V est compris dans une plage de 10 à 50.

3. Procédé de traitement de la poix, dans lequel le procédé d'analyse de la poix selon la revendication 1 ou la revendication 2 est mis en oeuvre en un point prédéterminé dans un système de fabrication de papier, puis, un agent de contrôle de la poix est ajouté en un point où une poix ayant une zone égale ou supérieure à une zone prédéterminée est présente dans l'image.
